(19) Europäisches Patentamt
European Patent Office
Office européen des brevets

(11) **EP 2 617 680 B1**

(12) **EUROPEAN PATENT SPECIFICATION**

(45) Date of publication and mention
of the grant of the patent:
**24.10.2018 Bulletin 2018/43**

(51) Int Cl.:
***B01J 29/035*** *(2006.01)*    ***B01J 37/00*** *(2006.01)*
***C07D 201/04*** *(2006.01)*    ***C01B 39/40*** *(2006.01)*

(21) Application number: **13151446.5**

(22) Date of filing: **16.01.2013**

(54) **Method for manufacturing zeolite and method for manufacturing epsilon-caprolactam**

Verfahren zur Herstellung von Zeolith und Verfahren zur Herstellung von Epsilon-Caprolactam

Procédé de fabrication d'une zéolite et procédé de fabrication d'epsilon-caprolactam

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.01.2012 JP 2012006795**

(43) Date of publication of application:
**24.07.2013 Bulletin 2013/30**

(73) Proprietor: **Sumitomo Chemical Company Limited Tokyo 104-8260 (JP)**

(72) Inventors:
• **Hoshino, Masahiro**
  **Ehime, 792-8521 (JP)**
• **Sugita, Keisuke**
  **Ehime, 792-8521 (JP)**
• **Kasai, Koji**
  **Ehime, 792-8521 (JP)**

(74) Representative: **Vossius & Partner Patentanwälte Rechtsanwälte mbB Siebertstrasse 3 81675 München (DE)**

(56) References cited:
**EP-A1- 1 065 167    EP-A2- 1 614 657 EP-A2- 2 157 080**

• **DATABASE WPI Week 201154 Thomson Scientific, London, GB; AN 2011-K12201 XP002697569, -& JP 2011 153047 A (SUMITOMO CHEM CO LTD) 11 August 2011 (2011-08-11)**
• **DATABASE WPI Week 201230 Thomson Scientific, London, GB; AN 2012-D86584 XP002697570, & JP 2012 066977 A (SUMITOMO CHEM CO LTD) 5 April 2012 (2012-04-05)**

EP 2 617 680 B1

**Description**

BACKGROUND OF THE INVENTION

The present invention relates to a method for manufacturing a pentasil type zeolite. Moreover, the present invention also relates to a method for manufacturing ε-caprolactam from cyclohexanone oxime using the pentasil type zeolite as a catalyst.

Conventionally, a method of a Beckmann rearrangement reaction of cyclohexanone oxime in a gaseous phase using a zeolite as a solid catalyst has been proposed as one of methods for manufacturing ε-caprolactam (see, for example, JP 2-250866 A and JP 2-275850 A). As such a method for manufacturing a zeolite, there has been proposed a method of subjecting a crystal obtained by a hydrothermal synthesis reaction of a silicon compound to a contact processing with an aqueous solution containing ammonia and an ammonium salt (JP 5-170732 A, JP 2001-72411 A, JP 2003-176125 A, JP 2004-75518 A, JP 2006-16220 A).

Further, it was proposed to produce a zeolite by firing a crystal obtained by hydrothermal synthesis of a silicon compound, subjecting the crystal to contact treatment with an aqueous solution containing ammonia and/or an ammonium salt, and heat treating the crystal at 500 to 700°C (JP 2011-153047A).

SUMMARY OF THE INVENTION

However, in the conventional methods mentioned above, an aqueous solution collected after the contact processing with an aqueous solution containing ammonia and an ammonium salt will contain ammonia and an ammonium salt, but there is no specific disclosure for a method of using repeatedly ammonia and/or an ammonium salt collected after the contact processing. Accordingly, an object of the present invention is to provide an industrially advantageous method for manufacturing a zeolite by effectively using ammonia and/or an ammonium salt collected after the contact processing so that an aqueous solution containing such ammonia and an ammonium salt can be used repeatedly. In addition, another object of the present invention is to provide a method for manufacturing ε-caprolactam in a good selectivity by reacting cyclohexanone oxime in a good conversion rate using as a catalyst the zeolite obtained by such a method.

As a result of intensive studies for the purpose of achieving the above objects, the present inventors have completed the present invention.

The present invention is as defined in the appended claims.

That is, the present invention comprises the following constitution.

[1] A method for manufacturing a pentasil type zeolite, comprising the following steps (1) to (4) of:

(1) conducting hydrothermal synthesis reaction of at least one silicon compound selected from the group consisting of amorphous silica, alkali silicate, and tetraalkyl orthosilicate at a temperature of 80 to 160°C and a pressure in the range of 0.10 to 1.0 MPa to provide a crystal;

(2) subjecting the crystal obtained by the hydrothermal synthesis reaction of at least one silicon compound to a contact processing with an aqueous solution containing ammonia in an amount of 2 to 30% by weight and an ammonium salt in an amount of 0.001 to 1 mol relative to 1 mol of ammonia, wherein the temperature of the contact processing with the aqueous solution is 50 to 250°C, and the time required for the contact processing is 0.1 to 10 hours;

(3) mixing the aqueous solution obtained after the contact processing in the step (2), with at least one selected from the group consisting of ammonia, an ammonium salt, and water, thereby to obtain an aqueous solution containing ammonia and an ammonium salt in which a silicon concentration is adjusted to 100 ppm by weight or less; and

(4) recycling the aqueous solution containing ammonia and an ammonium salt obtained in the step (3) into the step (2).

[2] The method according to [1], wherein in the step (3), a silicon component is removed from the aqueous solution obtained after the contact processing in the step (2), and then the aqueous solution is subjected to mixing with at least one selected from the group consisting of ammonia, an ammonium salt, and water.

[3] The method according to [1], wherein the step (3) is the following step (3') comprising removing ammonia from the aqueous solution obtained after the contact processing in the step (2), removing the silicon component from the aqueous solution, and then mixing the aqueous solution with ammonia, thereby to obtain an aqueous solution containing ammonia and an ammonium salt in which a silicon concentration is adjusted to 100 ppm by weight or less, and the aqueous solution containing ammonia and an ammonium salt obtained in the step (3') is subjected to the step (4).

[4] The method according to [2] or [3], wherein the silicon component is removed by separating it by filtration or sedimentation separation.

[5] The method according to any one of [1] to [4], wherein the crystal is calcined and then subjected to the contact processing in the step (2).

[6] The method according to any one of [1] to [5], wherein the at least one silicon compound in the step (2) is tetraalkyl orthosilicate.

[7] A method for manufacturing ε-caprolactam, comprising manufacturing a pentasil type zeolite according to the method described in any one of [1] to [6] and subjecting cyclohexanone oxime to a Beckmann rearrangement reaction in a gaseous phase in the presence of the zeolite.

According to the present invention, a zeolite can be industrially advantageously manufactured because ammonia and/or an ammonium salt collected in the manufacturing of the zeolite is effectively used, and their repeated use is possible as an aqueous solution containing ammonia and an ammonium salt. In addition, it is possible to suppress a decrease in catalytic activity caused by repeated use of an aqueous solution containing ammonia and an ammonium salt derived from ammonia and/or an ammonium salt collected during the manufacturing, and thus ε-caprolactam can be manufactured in a good selectivity by subjecting cyclohexanone oxime to a Beckmann rearrangement reaction in a gaseous phase in the presence of the zeolite obtained by such a method and thereby reacting cyclohexanone oxime in a good conversion rate.

DETAILED DESCRIPTION OF THE PREFERRED EMBODIMENTS

Hereinafter, the present invention will be described in detail. A zeolite, as a target to be manufactured in the present invention, includes silicon and oxygen as elements constituting a skeleton thereof and may be crystalline silica having a skeleton substantially constituted with silicon and oxygen, or may be a crystalline metallosilicate or the like, further containing other elements as elements constituting a skeleton thereof. In the case of crystalline metallosilicate or the like, other elements that may exist in addition to silicon and oxygen include, for example, Be, B, Al, Ti, V, Cr, Fe, Co, Ni, Cu, Zn, Ga, Ge, Zr, Nb, Sb, La, Hf, Bi, and the like, and two or more of them may be contained if necessary. Further, an atomic ratio of silicon to these elements is preferably 5 or more, and more preferably 500 or more.

Although various structures are known for the above-mentioned zeolite, a zeolite having a pentasil type structure is used in the present invention, and a zeolite having an MFI structure is especially preferable. Among the zeolites having an MFI structure, silicalite-1 is preferable. The silicalite-1 that is an MFI-zeolite having high silicate properties can be synthesized by hydrothermal synthesis of a mixture of tetraalkyl orthosilicate, water, and tetra-n-propylammonium hydroxide under an autogenous pressure.

In the present invention, the step (2) comprises a contact processing of a crystal obtained by a hydrothermal synthesis reaction of a silicon compound, using an aqueous solution containing ammonia and an ammonium salt.

The method of a hydrothermal synthesis reaction in the step (1) is not particularly limited and a known method can be appropriately employed for the reaction. For example, the hydrothermal synthesis reaction can be carried out by filling a mixture containing a silicon compound and water in a reaction vessel such as an autoclave and stirring the mixture in a sealed state under the temperature conditions described above . The silicon compound is selected from the group consisting of

amorphous silica such as colloidal silica, silica gel and fumed silica; alkali silicate such as sodium silicate and potassium silicate; and tetraalkyl orthosilicate such as tetramethyl orthosilicate, tetraethyl orthosilicate, tetrapropyl orthosilicate and tetrabutyl orthosilicate, and two or more of them can also be used if necessary. Among them, tetraalkyl orthosilicate is preferable, and tetraethyl orthosilicate is more preferable.

According to the structure of the desired zeolite, a structure-directing agent or the like is added to the mixture mentioned above. The structure-directing agent (template) refers to an organic compound that may be utilized to form a zeolite structure. The structure-directing agent is able to form a precursor of the zeolite structure by organizing polysilicic acid ions and polymetallosilicic acid ions around it (see, Science and Engineering of Zeolite (in Japanese), published by Kodansha Scientific Ltd., (2000), p.33-34). When the structure-directing agent is used in the hydrothermal synthesis reaction in the step (1), it includes a quaternary ammonium compound, an alkyl amine, and the like, among which a quaternary ammonium compound is preferable. Among the quaternary ammonium compound, tetraalkyl ammonium hydroxide and tetraalkyl ammonium halide are preferable, and tetraalkyl ammonium hydroxide is more preferable. The tetraalkyl ammonium hydroxide may act as a structure-directing agent or a base, and tetramethyl ammonium hydroxide and tetrapropyl ammonium hydroxide are preferably used and tetrapropyl ammonium hydroxide is more preferably used. When tetraalkyl ammonium hydroxide is used as the structure-directing agent in the step (1), a molar ratio of tetraalkylammonium ions to silicon in the mixture of a silicon compound, water, and tetraalkyl ammonium hydroxide is preferably 0.1 to 0.6, and more preferably 0.2 to 0.5.

The molar ratio of water to silicon in the mixture to be subjected to the hydrothermal synthesis reaction in the step (1) is preferably 5 to 100, and more preferably 10 to 60.

In the hydrothermal synthesis reaction in the step (1), the molar ratio of hydroxide ions to silicon in the mixture to be subjected to the hydrothermal synthesis reaction is adjusted to preferably 0.1 to 0.6, and more preferably 0.2 to 0.5.

When the mixture as described above is prepared, other components in addition to the silicon compound, water and the structure-directing agent may be used as raw materials if necessary. For example, a basic compound such as sodium hydroxide or potassium hydroxide may be mixed in order to adjust a concentration of hydroxide ions in the mixture. Also, for example, when tetraalkylammonium hydroxide is used as the structure-directing agent, a tetraalkylammonium salt such as tetraalkylammonium bromide may be mixed in order to adjust a concentration of tetraalkylammonium ions in the mixture. When a zeolite containing elements other than silicon and oxygen described above is prepared, a compound containing an element other than silicon and oxygen may be mixed. With respect to elements other than silicon and oxygen that were previously exemplified, when a compound containing these elements is mixed, a molar ratio of silicon to these elements in the mixture is preferably adjusted to 5 or more, and more preferably to 500 or more.

The content of silicon contained in the mixture of the step (1) can be determined, for example, by an inductively coupled plasma (ICP) optical emission spectrometry. In the case where tetraalkylammonium hydroxide is used as the structure-directing agent, a content of the tetraalkylammonium ions in the mixture can be determined by, for example, an ion chromatography. In addition, a content of hydroxide ions in the mixture can be determined by subtracting a content of basic ions other than hydroxide ions that may be contained as impurities in the structure-directing agent, from a total content of basic ions determined by neutralization titration with an acid (for example, 0.2 N hydrochloric acid).

In the step (1), the temperature in the hydrothermal synthesis reaction is 80 to 160°C, and preferably 100 to 140°C. Further, the reaction time required for the hydrothermal synthesis reaction is preferably 1 to 200 hours, and more preferably 12 to 72 hours. The pressure in the hydrothermal synthesis reaction is in the range of 0.10 to 1.0 MPa, and preferably in the range of 0.11 to 0.50 MPa (as absolute pressure).

As a method for separating a crystal from a reaction mixture containing the crystal obtained in the hydrothermal synthesis reaction of the step (1), it includes, for example, filtration, centrifugation, decantation and the like, but among them, filtration is preferably performed. As a filtration method, a membrane separation method using a microfiltration membrane (MF membrane) or an ultrafiltration membrane (UF membrane) is preferable. The material and pore size of such MF membrane or UF membrane are appropriately set. The filtration manner may include a cross-flow filtration manner and a dead-end filtration manner, but the cross-flow filtration manner is preferable from the viewpoint that washing can be easily and efficiently performed. In addition, the filtration manner may be an external pressure filtration manner or an inner pressure filtration manner. The filtration may be performed by any of a pressurized filtration or a suction filtration, but the pressurized filtration is preferable. The pressure in the filtration is appropriately set, and the filtration may be carried out by any of a constant flow filtration to perform the filtration while keeping an amount of filtrate constant or a constant pressure filtration to perform the filtration while keeping the transmembrane pressure difference constant. Because the filtrate obtained by the filtration contains usually silicic acid and oligomers thereof as an effective component and the unreacted structure-directing agent, and the like, when the structure-directing agent is used, at least a portion of the filtrate is mixed as a raw material in the hydrothermal synthesis reaction of the step (1) so that recycling use becomes possible. The crystal separated from the reaction mixture may be collected as a slurry and a content ratio of the crystal contained in the slurry is appropriately set.

The crystal separated from the reaction mixture is preferably subjected to a washing treatment. The washing treatment includes, for example, (A) a washing filtration method with water in a cross flow manner by an addition of water to the crystal; (B) a washing filtration method with water in a dead-end filtration manner by an addition of water to the crystal; (C) a method of mixing the crystal with water, stirring the mixture, and separating the supernatant by decantation; and the like. Among the methods (A) to (C), the method (A) is preferable from the viewpoint that washing can be performed easily and efficiently. When the washing filtration is carried out, a membrane separation method using an MF membrane or a UF membrane is preferable as such a washing filtration method. The material and pore size of such MF membrane or UF membrane are appropriately set. The washing filtration manner may include an external pressure filtration manner and an inner pressure filtration manner. The washing filtration may be performed by any of a pressurized filtration or a suction filtration, but the pressurized filtration is preferable. The pressure in the washing filtration is appropriately set, and the filtration may be carried out by any of a constant flow filtration to perform the filtration while keeping the amount of filtrate constant or a constant pressure filtration to perform the filtration while keeping the transmembrane pressure difference constant. Because the wash filtrate obtained by the washing treatment, particularly the wash filtrate at the initial stage of the washing step includes usually silicic acid and oligomers thereof as the effective components and the unreacted structure-directing agent, and the like, when the structure-directing agent is used, at least a portion of the wash filtrate is mixed as a raw material in the hydrothermal synthesis reaction of the step (1) so that its recycling use becomes possible. When tetraalkyl ammonium hydroxide is used as the structure-directing agent, the washing treatment is preferably carried out in such a manner that the pH at 25°C of the wash filtrate obtained after washing with water becomes 7 to 9.5. The crystal after the washing treatment may be collected as a slurry and the content of the crystal contained in the slurry is appropriately set.

The crystal separated from the reaction mixture is preferably calcined. In the case where the washing treatment is carried out, the crystal separated from the reaction mixture may be calcined following a washing treatment. When the hydrothermal synthesis reaction is performed using a structure-directing agent, the structure-directing agent is incorporated into the crystal obtained, but such a structure-directing agent that has been incorporated into the crystal is removed by this calcination. Calcination is usually carried out under an oxygen-containing gas atmosphere, for example, under an air atmosphere or a mixed gas atmosphere of air and oxygen, at a temperature of 400 to 600°C. In addition, calcination may be performed under an inert gas atmosphere such as nitrogen, and the like before and after such calcination under an oxygen-containing gas atmosphere.

The crystal may be dried as needed before calcination. The drying method is not particularly limited, and a conventional method usually employed in the art, such as evaporation to dryness method, spray-drying method, drum drying method, flash drying method, and the like may be adopted. In addition, the drying conditions may be appropriately set.

The crystal separated from the reaction mixture is subjected to a contact processing with an aqueous solution containing ammonia and an ammonium salt after optional calcination. By performing the contact processing with a specific aqueous solution in this manner, it is possible to manufacture a zeolite having an improved activity and life as the catalyst. Incidentally, other processing as needed, for example, contact with water vapor, may be applied to the crystal after calcination, prior to the contact processing with this aqueous solution, so that mechanical strength of the finally obtained zeolite can be improved.

When the contact processing with water vapor is carried out, the contact temperature is preferably 30 to 200°C, and more preferably 50 to 150°C. The contact time is usually 5 minutes to 72 hours. Such a contact processing may be repeated as needed. The contact processing method includes, for example, a method where water vapor flows through a column packed with the crystal after calcination; a method to retain the crystal after calcination under an atmosphere of water vapor; and the like. A basic gas such as ammonia and methylamine, an oxygen-containing gas, or an inert gas may coexist together with the water vapor. If the basic gas is allowed to coexist, such gas is used usually in 0.01 to 1 mol, preferably 0.1 to 0.5 mol, relative to 1 mol of water vapor. The crystal after such processing may be washed with an acid such as hydrochloric acid, or water, and when the crystal is washed with an acid, it may be further washed with water. The crystal after contacting with water vapor can also be further calcined as needed.

An ammonia concentration in the aqueous solution containing ammonia and an ammonium salt is 2 to 30% by weight, and preferably 5 to 25% by weight. Examples of the ammonium salt contained in the aqueous solution include ammonium chloride, ammonium sulfate, ammonium nitrate, ammonium acetate, ammonium phosphate, ammonium carbonate, ammonium fluoride, ammonium bromide, and the like, among which ammonium nitrate and ammonium acetate are preferable. A content of the ammonium salt in the aqueous solution is 0.001 to 1 mol, and preferably 0.01 to 0.1 mol, relative to 1 mol of ammonia.

The aqueous solution may contain other components other than ammonia and an ammonium salt, and examples of other components include a quaternary ammonium compound, a lower alkyl amine, and the like, and optionally two or more kinds thereof. Among them, the quaternary ammonium compound is preferably contained. If at least one selected from the group consisting of a quaternary ammonium compound and a lower alkylamine is contained in the aqueous solution, a content of at least one selected from the group consisting of a quaternary ammonium compound and a lower alkylamine is usually 0.0000001 to 1 mol, and preferably 0.000001 to 0.1 mol, relative to 1 mol of ammonia. If the quaternary ammonium compound and lower alkyl amine are contained, a total content thereof may be in the range described above. The aqueous solution should adjust a content of ammonia, the ammonium salt and other components as needed in the aqueous solution so that the pH becomes usually 9 or more, preferably 9 to 13.

If a quaternary ammonium compound is contained in the aqueous solution, examples of the quaternary ammonium compound include hydroxides or halides of various quaternary ammoniums such as tetramethyl ammonium, tetraethyl ammonium, n-propyltrimethyl ammonium, tetra-n-propyl ammonium, tetra-n-butyl ammonium, triethylmethyl ammonium, tri-n-propylmethyl ammonium, tri-n-butylmethyl ammonium, benzyltrimethyl ammonium and dibenzyldimethyl ammonium), and two or more of them can be used as needed. In particular, tetra-n-propyl ammonium compounds are preferable, and tetra-n-propyl ammonium hydroxide and tetra-n-propylammonium bromide are more preferable.

When a lower alkylamine is contained in the aqueous solution, this lower alkylamine may be monoalkylamines, dialkylamines, or a trialkylamines, and two or more kinds thereof can also be used as needed. Usually, a compound represented by the following formula (I):

$$R^1R^2R^3N \qquad (I)$$

(wherein $R^1$, $R^2$, and $R^3$ each independently represents a hydrogen atom, an alkyl group having 1 to 4 carbon atoms, or an alkenyl group having 1 to 4 carbon atoms, provided that $R^1$, $R^2$, and $R^3$ are not hydrogen atoms at the same time) is preferably used.

Examples of the lower alkylamine represented by the formula (I) include alkylamines such as monomethylamine, monoethylamine, monopropylamine, monobutylamine, dimethylamine, diethylamine, dipropylamine, dibutylamine, trimethylamine, triethylamine, tripropylamine and tributylamine, allylamines such as monoallylamine, diallylamine and triallylamine, and the like, and two or more of them may be used if necessary. Among them, tripropylamine is preferable.

The contact processing with the aqueous solution may be performed in a batch manner or in a continuation manner. For example, the crystal may be dipped in the aqueous solution and stirred in a stirred tank, or the aqueous solution may be allowed to flow through a tubular container filled with the crystal.

The temperature of the contact processing with the aqueous solution is usually 50 to 250°C, preferably 50 to 200°C, and more preferably 60 to 150°C, and the time required for the contact processing is usually 0.1 to 10 hours. The amount of the aqueous solution to be used is usually 100 to 5000 parts by weight, relative to 100 parts by weight of the crystal.

The zeolite after the contact processing with the aqueous solution is subjected to a processing such as washing with water, drying, and the like as needed. The contact processing with the aqueous solution may be carried out in a plural time if necessary.

The zeolite after the contact processing with the aqueous solution may be subjected to a heat treatment, if necessary. The temperature of the heat treatment is preferably 400 to 700°C, and the time required for the heat treatment is preferably 0.1 to 100 hours.

The heat treatment may be carried out under an atmosphere of an oxygen-containing gas or may be carried out under an atmosphere of an inert gas such as nitrogen. The oxygen-containing gas and inert gas may contain water vapor. In addition, the heat treatment may be carried out in multiple stages under an atmosphere of an oxygen-containing gas or an inert gas. The heat treatment may be performed in a fluidized bed manner or in a fixed bed manner.

In the present invention, the step (3) comprises mixing the aqueous solution obtained after the contact processing in the step (2), with at least one selected from the group consisting of ammonia, an ammonium salt, and water, thereby to obtain an aqueous solution containing ammonia and an ammonium salt in which a silicon concentration is adjusted to 100 ppm by weight or less. A method for separating the crystal and the aqueous solution after the contact processing is appropriately selected according to the manner of the contact processing and is preferably performed by, for example, filtration, centrifugation, decantation, and the like, among which filtration is preferable. The aqueous solution that was separated and collected will contain usually silica and a silicic acid compound dissolved by the contact processing, and a silicon component (compounds containing silicon and oxygen) derived from the crystal, in addition to ammonia and the ammonium salt. When the aqueous solution that was separated and collected is recycled into the contact processing of the step (2), the catalytic activity of the obtained zeolite may be affected depending on the content of the silicon component. Accordingly, by mixing the aqueous solution containing ammonia and an ammonium salt, obtained after the contact processing in the step (2), with at least one selected from the group consisting of ammonia, an ammonium salt, and water in such a manner that the silicon concentration is adjusted to 100 ppm by weight or less in the resulting aqueous solution containing ammonia and an ammonium salt, it becomes possible to obtain a zeolite having an excellent catalytic activity, even if the ammonia and/or an ammonium salt collected in the aqueous solution obtained after the contact processing in the step (2) are repeatedly used. The silicon concentration in the aqueous solution containing ammonia and an ammonium salt obtained in the step (3) is preferably 80 ppm by weight or less, and more preferably 60 ppm by weight or less. The aqueous solution may contain other components such as quaternary ammonium compounds and lower alkylamines which may be used in the above contact processing.

As a method to obtain an aqueous solution containing ammonia and an ammonium salt in the step (3) in which a silicon concentration is adjusted to 100 ppm by weight or less, specifically, the mixing ratio of the aqueous solution after the contact processing with at least one selected from the group consisting of ammonia, an ammonium salt, and water may be adjusted in consideration of the silicon concentration in the aqueous solution after the contact processing. Further, in consideration of the silicon concentration in the aqueous solution after the contact processing, the silicon component is removed from the aqueous solution after the contact processing, and the resulting aqueous solution is mixed with at least one selected from the group consisting of ammonia, an ammonium salt, and water, so that an aqueous solution containing ammonia and an ammonium salt in which a silicon concentration is adjusted to 100 ppm by weight or less may be obtained.

When the silicon component is removed from the aqueous solution after the contact processing, the silicon component from the aqueous solution after the contact processing is removed by, for example, filtration, centrifugation, sedimentation separation, and the like, among which filtration or sedimentation separation is preferable. If necessary, crystallization, concentration, neutralization, and the like for precipitating the silicon component is applied before an operation such as filtration, centrifugation, sedimentation separation, and the like. An aqueous solution with a reduced silicon concentration can be collected by removing the silicon component from the aqueous solution after the contact processing. When the filtration is carried out, a membrane separation method using an MF membrane or a UF membrane is preferable as such a filtration method. The material and pore size of such MF membrane or UF membrane are appropriately set. The filtration manner may be an external pressure filtration manner and an inner pressure filtration manner. The filtration may be performed by any of a pressurized filtration or a suction filtration, but a pressurized filtration is preferable. The pressure in the filtration is appropriately set, and the filtration may be carried out by any of a constant flow filtration to perform the filtration while keeping the amount of filtrate constant or a constant pressure filtration to perform the filtration while keeping the transmembrane pressure difference constant. In the aqueous solution obtained after removal of the silicon component from the aqueous solution after the contact processing, the silicon concentration is preferably 100 ppm by weight or less. If the silicon concentration in the aqueous solution obtained after removal of the silicon component exceeds 100 ppm by weight, an aqueous solution containing ammonia and an ammonium salt in which a silicon concentration is adjusted to 100 ppm by weight or less may be obtained by adjusting the mixing ratio of the obtained aqueous solution with at least one selected from the group consisting of ammonia, an ammonium salt, and water in consideration of the silicon concentration in the aqueous solution after removal of the silicon component.

The step (3) may comprise the step (3') of removing ammonia from the aqueous solution obtained after the contact processing in the step (2), removing the silicon component from the aqueous solution, and mixing the aqueous solution with ammonia so that an aqueous solution containing ammonia and an ammonium salt in which a silicon concentration is adjusted to 100 ppm by weight or less is obtained. By removing ammonia in advance, the operability of removal processing for the silicon component is improved. The removal of ammonia from the aqueous solution after the contact processing is preferably performed by separating ammonia by distillation. The removal of the silicon component from the aqueous solution after removal of ammonia can be carried out in the same manner as in the removal of the silicon component from the aqueous solution after the contact processing described above. At the time of mixing with ammonia, an ammonia concentration, an ammonium salt concentration, and a silicon concentration in the aqueous solution may be adjusted by mixing with at least one selected from the group consisting of an ammonium salt and water as needed. In the aqueous solution obtained by removing the silicon component from the aqueous solution after removal of ammonia, the silicon concentration is preferably 100 ppm by weight or less. If the silicon concentration in the aqueous solution obtained after removal of the silicon component exceeds 100 ppm by weight, an aqueous solution containing ammonia and an ammonium salt in which a silicon concentration is adjusted to 100 ppm by weight or less may be obtained by adjusting the mixing ratio of the obtained aqueous solution, ammonia, and optionally at least one selected from the group consisting of an ammonium salt and water in consideration of the silicon concentration in the aqueous solution after the removal of the silicon component, and then mixing them. The separated ammonia may be appropriately purified and used for the preparation of an aqueous solution, which is to be recycled into the step (2), containing ammonia and an ammonium salt.

In the aqueous solution containing ammonia and an ammonium salt obtained in the step (3) or (3') in which a silicon concentration is adjusted to 100 ppm by weight or less, the ammonia concentration is preferably 2 to 30% by weight, and more preferably 5 to 25% by weight. The content of the ammonium salt in the aqueous solution is preferably 0.001 to 1 mol, and more preferably 0.01 to 0.1 mol, relative to 1 mol of ammonia.

The concentration of silicon in each aqueous solution in the step (3) or (3') can be determined by, for example, ICP optical emission spectrometry.

In the present invention, the step (4) comprises recycling an aqueous solution containing ammonia or an ammonium salt obtained in the step (3) or (3') into the step (2). At the time of recycling the aqueous solution containing ammonia and an ammonium salt into the step (2), other components such as quaternary ammonium compounds, lower alkylamines, and the like usable in the above contact processing may be mixed with the aqueous solution. The method for manufacturing a zeolite according to the present invention is carried out by including the steps (1) to (4) for the purpose of improving the activity and life in the obtained zeolite catalyst, which method makes it possible to effectively use ammonia and/or an ammonium salt collected in the contact processing of an aqueous solution containing ammonia and an ammonium salt and to use them repeatedly.

The zeolite as the catalyst may be used after molding according to a reaction vessel and the like to be used. In molding a zeolite, for example, an extrusion, compression, tablet, flow, rolling, or spray molding can be used, and the zeolite can be molded into a desired shape (for example, sphere, column, plate, ring, clover, and the like). The molding may be carried out after separation of the crystal in the step (1), or may be carried out after separation of the crystal following the contact processing in the step (3) or (3'). Further, when the washing treatment is performed in the step (1), the molding after the washing treatment may be carried out, or when the calcination is performed in the step (1), the molding may be carried out after such calcination. As for a zeolite molded article, a shape may be chosen depending on its use and a molding method may be chosen depending on its shape. For example, when the zeolite molded article is used as a fixed bed catalyst, a pellet-like molding such as column and cylinder is manufactured by an extrusion or tablet molding, and when the zeolite molded article is used as a catalyst for a fluidized bed reaction or a moving bed reaction, fine particle moldings such as spheres are manufactured by spray drying the slurry. When the molding is performed by spray drying, the content of the crystal contained in the slurry to be subjected to such a spray drying is appropriately set. In addition, this zeolite may comprise substantially only zeolite or may comprise zeolite and other components. For example, the zeolite may be those obtained by molding substantially only zeolite, those obtained by mixing a zeolite with binders and reinforcing materials, or those obtained by supporting it on a carrier. Moreover, the particle size of the zeolite molded article is usually 5 mm or less, preferably 3 mm or less. As the zeolite to be molded, it is preferable to use a zeolite having a primary particle diameter of 5 $\mu$m or less, and more preferably 1 $\mu$m or less.

The thus obtained zeolite can be used in various applications including a catalyst for organic synthesis reactions. Among them, such a zeolite can be used suitably as a catalyst for manufacturing $\varepsilon$-caprolactam by a Beckmann rearrangement reaction of cyclohexanone oxime in a gaseous phase.

As reaction conditions for Beckmann rearrangement, the reaction temperature is usually 250 to 500°C, preferably 300 to 450°C. The reaction pressure is usually 0.005 to 0.5 MPa, preferably 0.005 to 0.2 MPa. This reaction may be performed either in a fixed bed system or in a fluidized bed system. The rate of feed of cyclohexanone oxime as a raw material is usually 0.1 to 20 h$^{-1}$, preferably 0.2 to 10 h$^{-1}$, as a feeding rate of it (kg/h) per 1 kg of catalyst, that is, as a space velocity WHSV (h$^{-1}$).

Cyclohexanone oxime may, for example, either be independently introduced into a reaction system or may be introduced together with an inert gas, such as nitrogen, argon and carbon dioxide. In addition, the following methods are also effective, that is: a method by coexistence of ethers described in JP 2-250866 A; a method by coexistence of lower alcohols described in JP 2-275850 A; a method by coexistence of alcohols and/or ethers, and water described in JP 5-201965 A; a method by coexistence of ammonia described in JP 5-201966 A; and a method by coexistence of methylamine described in JP 6-107627 A.

In addition, cyclohexanone oxime may be, for example, those prepared by oximation of cyclohexanone with hydroxylamine or its salt, or those prepared by ammoximation of cyclohexanone with ammonia and hydrogen peroxide in the presence of a catalyst such as titano-silicate, and the like.

Further, the Beckmann rearrangement reaction may be carried out in combination with an operation of calcining a catalyst under an atmosphere of an oxygen-containing gas such as air, and the like. This calcining treatment of the catalyst enables removing by combustion of carbonaceous materials deposited on the catalyst, and can increase a conversion of the cyclohexanone oxime and a persistency of selectivity of the $\varepsilon$-caprolactam. For example, when the reaction is performed in a fixed bed system, a method is suitably adopted wherein cyclohexanone oxime optionally together with other components is supplied into a fixed bed type reactor having a solid catalyst charged therein so that the reaction is performed, subsequently supply of cyclohexanone oxime is stopped, then calcination is carried out by supplying an oxygen-containing gas, and furthermore these reaction and calcination are repeated. When the reaction is performed in a fluidized bed system, a method is suitably adopted wherein while performing the reaction by supplying cyclohexanone oxime optionally with other components into a fluidized bed reactor having a solid catalyst being fluidized therein, the solid catalyst is withdrawn continuously or intermittently from the reactor and returned into the reactor again after calcined in a calcination furnace.

Further, as post-treatment operations of the reaction mixture obtained by the reaction described above, publicly known methods can be appropriately employed, and for example, after condensation by cooling the gas produced by the reaction, $\varepsilon$-caprolactam can be separated by performing operations of extraction, distillation, crystallization, and the like.

EXAMPLES

Hereinafter, Examples of the present invention will be described, but the present invention is not limited thereto. In addition, the space velocity WHSV (h$^{-1}$) of cyclohexanone oxime was calculated by dividing a feeding rate (kg/h) of cyclohexanone oxime by a catalyst weight (kg). Analysis of cyclohexanone oxime and $\varepsilon$-caprolactam was conducted using a gas chromatography, and a conversion of cyclohexanone oxime and a selectivity of $\varepsilon$-caprolactam were calculated by the following equations, respectively, where the number of moles of supplied cyclohexanone oxime was defined as X, the number of moles of unreacted cyclohexanone oxime as Y, and the number of moles of formed $\varepsilon$-caprolactam as Z.

- $$\text{Conversion of cyclohexanone oxime (\%)} = [(X - Y)/X] \times 100$$

- $$\text{Selectivity of } \varepsilon\text{-caprolactam (\%)} = [Z/(X - Y)] \times 100$$

Example 1

Manufacture of zeolite [1]

Hydrothermal synthesis

Into a 30L-stainless steel autoclave were added 100 parts by weight of tetraethyl orthosilicate $[Si(OC_2H_5)_4]$, 57.4 parts by weight of a 40% (by weight) aqueous tetra-n-propylammonium hydroxide solution, 0.36 parts by weight of a 48% (by weight) aqueous potassium hydroxide solution and 279 parts by weight of water, and the mixture was stirred vigorously at room temperature for 120 minutes, after which time a hydrothermal synthesis reaction was performed with stirring at 105°C and 300 rpm for 48 hours. The obtained reaction mixture was subjected to a cross flow filtration to separate a concentrate containing a crystal and a filtrate. The concentrate containing the obtained crystal was washed with water and filtered in a cross flow manner and washed repeatedly until the pH of the wash filtrate becomes around 9. After washing, a slurry containing crystal was collected.

Molding, calcination, and water vapor treatment

The slurry containing crystal obtained in the above Hydrothermal synthesis was spray-dried with an atomizer type spray dryer to form fine particles. The average inlet temperature and the average outlet temperature of the spray dryer were 210°C and 90°C, respectively. Using a rotary kiln, the white fine particles obtained were calcined at 530°C in a nitrogen flow for one hour, and further calcined at 530°C in a mixed gas flow of nitrogen and air [nitrogen : air = 4 : 1 (volume ratio)] for one hour, thereby to obtain a white calcined product. Then, 10 parts by weight of the calcined product obtained above was placed in a Petri dish and hanged in a 1-liter autoclave containing 100 parts by weight of water. After closing the lid, the autoclave was placed in a thermostatic chamber at 80°C and allowed to stand for 3 hours. After removing the autoclave from the chamber, it was allowed to cool to 20°C.

Contact processing with aqueous solution (A)

The solid (8.74 parts by weight) obtained in the Molding, calcination, and water vapor treatment described above was placed in an autoclave, and 242.98 parts by weight of a mixed solution (A) of 96.14 parts by weight of a 7.5% (by weight) aqueous ammonium nitrate solution and 146.84 parts by weight of a 25% (by weight) aqueous ammonia solution were added thereto. The mixture was stirred at 90°C for one hour, and filtered to separate a solid and collect a filtrate. This solid was further treated twice with a mixture of an aqueous ammonium nitrate solution and an aqueous ammonia solution in the same manner as described above, and then washed and dried to obtain a zeolite (A).

Manufacture of ε-caprolactam [1]

The zeolite (A) 0.375 g obtained above was charged into a tubular reactor made of quartz glass having an inner diameter of 1 cm to form a catalyst layer, and this was preheated at 350°C for one hour in a nitrogen flow of 4.2 L/h. Subsequently, after lowering the temperature of the catalyst layer to 325°C in a nitrogen flow of 4.2 L/h, a mixture of vaporized cyclohexanone oxime/methanol = 1/1.8 (weight ratio) was supplied to the tubular reactor at a feeding rate of 8.4 g/h (WHSV=8 $h^{-1}$ of cyclohexanone oxime) to be reacted. A reaction gas after 20.00 hours to 20.25 hours from the start of the reaction was sampled and analyzed using gas chromatography. The conversion of cyclohexanone oxime was 99.9%, and the selectivity of ε-caprolactam was 97.0%.

Preparation of recycled aqueous solution [1]

A mixture (997.8 g) of the filtrate collected by the second treatment with a mixture (A) of an aqueous ammonium nitrate solution and an aqueous ammonia solution in the above Contact processing with aqueous solution (A) and the filtrate collected by the third treatment with a mixture (A) of an aqueous ammonium nitrate solution and an aqueous ammonia solution was concentrated by distilling off the ammonia and water under the conditions of 65°C and 30 Torr to obtain a concentrate of 399.1 g. The solid silicon component that was precipitated in the obtained concentrate was removed by filtration to obtain an aqueous solution (B) containing ammonium nitrate (content of ammonium nitrate: 7.5% by weight). The silicon content of the obtained aqueous solution (B) containing ammonium nitrate was determined to be 60 ppm by weight by analysis using an ICP optical emission spectrometry device ("SPS 4000", manufactured by SII NanoTechnology Inc.). The obtained aqueous solution (B) (96.14 parts by weight) containing ammonium nitrate was mixed with a 25% (by weight) aqueous ammonia solution (146.84 parts by weight) to obtain 242.98 parts by weight of an aqueous solution (C) containing ammonia and ammonium nitrate (silicon content: 24 ppm by weight).

Manufacture of zeolite [2]

Contact processing with aqueous solution (B)

The solid (8.74 parts by weight) obtained in the Molding, calcination, and water vapor treatment described above was placed in an autoclave, and the aqueous solution (C) (242.98 parts by weight) obtained in the Preparation of recycled aqueous solution [1] described above containing ammonia and ammonium nitrate was added thereto. The mixture was stirred at 90°C for one hour and filtered to separate a solid and collect a filtrate. This solid was further treated twice with the aqueous solution (C) containing ammonia and ammonium nitrate in the same manner as described above, and then washed and dried to obtain a zeolite (B).

Manufacture of ε-caprolactam [2]

The same procedure as in the Manufacture of ε-caprolactam [1] was performed, except that the zeolite (B) was used in place of the zeolite (A). The conversion of cyclohexanone oxime was 99.9% and the selectivity of ε-caprolactam was 96.6%.

Comparative Example 1

Manufacture of zeolite [3]

The zeolite (A) was obtained in the same manner as in Manufacture of zeolite [1] Hydrothermal synthesis , Molding, calcination, and water vapor treatment , and Contact processing with aqueous solution (A) of Example 1.

Preparation of recycled aqueous solution [2]

A mixture (445.48 g) of the filtrate collected by the second treatment with a mixture (A) of an aqueous ammonium nitrate solution and an aqueous ammonia solution in the above-mentioned Contact processing with aqueous solution (A) and the filtrate collected by the third treatment with a mixture (A) of an aqueous ammonium nitrate solution and an aqueous ammonia solution was concentrated by distilling off the ammonia and water under the conditions of 65°C and 30 Torr to obtain 178.19 g of an aqueous ammonium nitrate solution (D) (content of ammonium nitrate: 7.5% by weight). The silicon content of the obtained aqueous solution (D) containing ammonium nitrate was determined to be 810 ppm by weight by analysis using an ICP optical emission spectrometry device ("SPS 4000", manufactured by SII NanoTechnology Inc.). The obtained aqueous solution (D) (48.07 parts by weight) containing ammonium nitrate was mixed with a 7.5% (by weight) aqueous ammonium nitrate solution (48.07 parts by weight) and a 25% (by weight) aqueous ammonia solution (146.84 parts by weight) to obtain 242.98 parts by weight of an aqueous solution (E) containing ammonia and ammonium nitrate (silicon content: 160 ppm by weight).

Manufacture of zeolite [4]

Contact processing with aqueous solution (C)

The solid (8.74 parts by weight) obtained in the Molding, calcination, and water vapor treatment described above was placed in an autoclave, and the aqueous solution (E) (242.98 parts by weight) containing ammonia and ammonium nitrate obtained in the Preparation of recycled aqueous solution [2] described above was added thereto. The mixture was stirred at 90°C for one hour and filtered to separate a solid and collect a filtrate. This solid was further treated twice with the aqueous solution (E) containing ammonia and ammonium nitrate in the same manner as described above, and then washed and dried to obtain a zeolite (C).

Manufacture of ε-caprolactam [3]

The same procedure as in the Manufacture of ε-caprolactam [1] was performed, except that the zeolite (C) was used in place of the zeolite (A). The conversion of cyclohexanone oxime was 98.0% and the selectivity of ε-caprolactam was 96.6%.

**Claims**

1. A method for manufacturing a pentasil type zeolite, comprising the following steps (1) to (4) of:

(1) conducting hydrothermal synthesis reaction of at least one silicon compound selected from the group consisting of amorphous silica, alkali silicate, and tetraalkyl orthosilicate at a temperature of 80 to 160°C and a pressure in the range of 0.10 to 1.0 MPa to provide a crystal;
(2) subjecting the crystal obtained by the hydrothermal synthesis reaction of at least one silicon compound to a contact processing with an aqueous solution containing ammonia in an amount of 2 to 30% by weight and an ammonium salt in an amount of 0.001 to 1 mol relative to 1 mol of ammonia,
wherein the temperature of the contact processing with the aqueous solution is 50 to 250°C, and the time required for the contact processing is 0.1 to 10 hours;
(3) mixing the aqueous solution obtained after the contact processing in the step (2), with at least one selected from the group consisting of ammonia, an ammonium salt, and water, thereby to obtain an aqueous solution containing ammonia and an ammonium salt in which a silicon concentration is adjusted to 100 ppm by weight or less; and
(4) recycling the aqueous solution containing ammonia and an ammonium salt obtained in the step (3) into the step (2).

2. The method according to claim 1, wherein in the step (3), a silicon component is removed from the aqueous solution obtained after the contact processing in the step (2), and then the aqueous solution is subjected to mixing with at least one selected from the group consisting of ammonia, an ammonium salt, and water.

3. The method according to claim 1, wherein the step (3) is the following step (3') comprising removing ammonia from the aqueous solution obtained after the contact processing in the step (2), removing the silicon component from the aqueous solution, and then mixing the aqueous solution with ammonia, thereby to obtain an aqueous solution containing ammonia and an ammonium salt in which a silicon concentration is adjusted to 100 ppm by weight or less, and the aqueous solution containing ammonia and an ammonium salt obtained in the step (3') is subjected to the step (4).

4. The method according to claim 2 or 3, wherein the silicon component is removed by separating it by filtration or sedimentation separation.

5. The method according to any one of claims 1 to 4, wherein the crystal is calcined and then subjected to the contact processing in the step (2).

6. The method according to any one of claims 1 to 5, wherein the at least one silicon compound in the step (2) is tetraalkyl orthosilicate.

7. A method for manufacturing ε-caprolactam, comprising manufacturing a pentasil type zeolite according to the method described in any one of claims 1 to 6 and subjecting cyclohexanone oxime to a Beckmann rearrangement reaction in a gaseous phase in the presence of the zeolite.

**Patentansprüche**

1. Verfahren zur Herstellung von Zeolit vom Typ Pentasil, beinhaltend folgende Schritte (1) bis (4):

(1) Durchführen einer hydrothermalen Synthesereaktion von mindestens einer Silikonverbindung, gewählt aus der Gruppe, bestehend aus amorpher Silika, Alkalisilikat und Tetraalkylorthosilikat bei einer Temperatur von 80 bis 160°C und einem Druck in dem Bereich von 0,10 bis 1,0 MPa zum Bereitstellen eines Kristalls;
(2) Unterwerfen des durch die hydrothermale Synthesereaktion von mindestens einer Silikonverbindung erzielten Kristalls einer Kontaktverarbeitung mit einer wässrigen Lösung, welche Ammoniak in einer Menge von 2 bis 30 Gewichtsprozent und ein Ammoniumsalz in einer Menge von 0,001 bis 1 Mol bezogen auf 1 Mol Ammoniak enthält,
wobei die Temperatur der Kontaktverarbeitung mit der wässrigen Lösung 50 bis 250°C beträgt, und die für die Kontaktverarbeitung notwendige Zeit 0,1 bis 10 Stunden beträgt;
(3) Mischen der nach der Kontaktverarbeitung in Schritt (2) erzielten wässrigen Lösung mit mindestens einem Element, gewählt aus der Gruppe, bestehend aus Ammoniak, einem Ammoniumsalz und Wasser, um dadurch eine wässrige Lösung zu erzielen, welche Ammoniak und ein Ammoniumsalz enthält, in welcher eine Silikonkonzentration auf 100 Gewichts-ppm oder darunter justiert ist; und

(4) Recyceln in Schritt (2) der in Schritt (3) erzielten wässrigen Lösung, welche Ammoniak und ein Ammoniumsalz enthält.

2. Verfahren nach Anspruch 1, bei welchem in Schritt (3) eine Silikonkomponente aus der wässrigen Lösung entfernt wird, welche nach der Kontaktverarbeitung in Schritt (2) erzielt wurde, und bei welchem danach die wässrige Lösung einem Mischen mit mindestens einem Element unterworfen wird, gewählt aus der Gruppe, bestehend aus Ammoniak, einem Ammoniumsalz und Wasser.

3. Verfahren nach Anspruch 1, bei welchem Schritt (3) der folgende Schritt (3') ist, beinhaltend Entfernen von Ammoniak aus der wässrigen Lösung, welche nach der Kontaktverarbeitung in Schritt (2) erzielt wurde, Entfernen der Silikonkomponente aus der wässrigen Lösung, und danach, Mischen der wässrigen Lösung mit Ammoniak, um dadurch eine wässrige Lösung zu erzielen, welche Ammoniak und ein Ammoniumsalz enthält, in welcher eine Silikonkonzentration auf 100 Gewichts-ppm oder darunter justiert ist, und wobei die Ammoniak und ein Ammoniumsalz enthaltende wässrige Lösung, welche in Schritt (3') erzielt wurde, Schritt (4) unterworfen wird.

4. Verfahren nach Anspruch 2 oder 3, bei welchem die Silikonkomponente durch Abscheiden der Komponente durch Filterung oder Sedimentierungs-Abscheidung entfernt wird.

5. Verfahren nach einem der Ansprüche 1 bis 4, bei welchem das Kristall kalziniert und danach der Kontaktverarbeitung in Schritt (2) unterworfen wird.

6. Verfahren nach einem der Ansprüche 1 bis 5, bei welchem die mindestens eine Silikonverbindung in Schritt (2) Tetraalkylorthosilikat ist.

7. Verfahren zur Herstellung von $\varepsilon$-Caprolactam, beinhaltend Herstellen eines Zeolits vom Typ Pentasil nach dem in einem der Ansprüche 1 bis 6 beschriebenen Verfahren, und Unterwerfen von Cyclohexanonoxim einer Beckmann-Umlagerungsreaktion in einer gasförmigen Phase unter Vorhandensein des Zeolits.

**Revendications**

1. Procédé de fabrication d'une zéolite de type pentasil, comprenant les étapes (1) à (4) ci-dessous consistant à :

(1) mettre en oeuvre une réaction de synthèse hydrothermale d'au moins un composé à base de silicium sélectionné parmi le groupe constitué de silice amorphe, silicate alcalin, et orthosilicate de tétraalkyle à une température comprise entre 80 et 160°C et à une pression comprise entre 0,10 et 1,0 MPa afin de fournir un cristal ;

(2) soumettre le cristal obtenu grâce à la réaction de synthèse hydrothermale d'au moins un composé à base de silicium à un traitement par contact avec une solution aqueuse contenant de l'ammoniac en une quantité comprise entre 2 et 30 % en poids et un sel d'ammonium en une quantité comprise entre 0,001 et 1 mole pour 1 mole d'ammoniac,

dans lequel la température du traitement par contact avec la solution aqueuse est comprise entre 50 et 250°C, et le temps requis pour le traitement par contact est compris entre 0,1 et 10 heures ;

(3) mélanger la solution aqueuse, obtenue après le traitement par contact de l'étape (2), avec au moins un élément sélectionné parmi le groupe constitué d'ammoniac, un sel d'ammonium, et eau, afin d'obtenir ainsi une solution aqueuse contenant de l'ammoniac et un sel d'ammonium au sein de laquelle une concentration en silicium est ajustée de manière à être inférieure ou égale à 100 ppm en poids ; et

(4) recycler la solution aqueuse contenant de l'ammoniac et un sel d'ammonium obtenue dans l'étape (3) vers l'étape (2).

2. Procédé selon la revendication 1, dans lequel, lors de l'étape (3), un composant silicium est retiré de la solution aqueuse obtenue après le traitement par contact de l'étape (2), et la solution aqueuse est ensuite soumise à un mélange avec au moins un élément sélectionné parmi le groupe constitué d'ammoniac, un sel d'ammonium, et eau.

3. Procédé selon la revendication 1, dans lequel l'étape (3) est l'étape (3') ci-dessous comprenant les étapes consistant à retirer de l'ammoniac de la solution aqueuse obtenue après le traitement par contact de l'étape (2), retirer le composant silicium de la solution aqueuse, et mélanger ensuite la solution aqueuse avec de l'ammoniac, afin d'obtenir ainsi une solution aqueuse contenant de l'ammoniac et un sel d'ammonium au sein de laquelle une concentration en silicium est ajustée de manière à être inférieure ou égale à 100 ppm en poids, et la solution aqueuse contenant de l'ammoniac et un sel d'ammonium obtenue lors de l'étape (3') est soumise à l'étape (4).

4. Procédé selon la revendication 2 ou 3, dans lequel le composant silicium est retiré grâce à une étape consistant à le séparer par filtration ou sédimentation.

5. Procédé selon l'une quelconque des revendications 1 à 4, dans lequel le cristal est calciné et soumis ensuite au traitement par contact de l'étape (2).

6. Procédé selon l'une quelconque des revendications 1 à 5, dans lequel le au moins un composé à base de silicium de l'étape (2) est un orthosilicate de tétraalkyle.

7. Procédé de fabrication de ε-caprolactame, comprenant les étapes consistant à fabriquer une zéolite de type pentasil conformément au procédé selon l'une quelconque des revendications 1 à 6 et soumettre l'oxime de la cyclohexanone à une réaction de réarrangement de Beckmann dans une phase gazeuse en présence de la zéolite.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2250866 A
- JP 2275850 A
- JP 5170732 A
- JP 2001072411 A
- JP 2003176125 A
- JP 2004075518 A
- JP 2006016220 A
- JP 2011153047 A
- JP 5201965 A
- JP 5201966 A
- JP 6107627 A

**Non-patent literature cited in the description**

- Science and Engineering of Zeolite. Kodansha Scientific Ltd, 2000, 33-34